# EUROPEAN PATENT SPECIFICATION

(11) **EP 3 913 043 B1**
(45) Date of publication and mention of the grant of the patent: **11.10.2023**
(21) Application number: 21175469.2
(22) Date of filing: 21.05.2021
(51) Int. Cl.: C12M 1/33, A61K 35/35, A61L 27/38, C12M 1/00

(54) **DEVICE AND METHOD FOR THE EXTRACTION OF STEM CELLS FROM ADIPOSE TISSUE**
VORRICHTUNG UND VERFAHREN ZUR EXTRAKTION VON STAMMZELLEN AUS FETTGEWEBE
DISPOSITIF ET PROCÉDÉ D'EXTRACTION DE CELLULES SOUCHES À PARTIR DE TISSUS ADIPEUX

(30) Priority: 21.05.2020 IT 202000011926
(43) Date of publication of application: 24.11.2021
(73) Proprietor: Università degli Studi di Bari "Aldo Moro", 70121 Bari (BA) (IT); MASMEC S.p.A., 70026 Modugno (IT)
(72) Inventor: GIORGINO, Francesco, 70121 BARI (IT); PERRINI, Sebastio, 70121 BARI (IT); LARIZZA, Pietro, 70026 MODUGNO (BA) (IT)
(74) Representative: Studio Torta S.p.A.

(56) References cited:
- US-A1- 2016 252 537
- US-A1- 2016 298 076
- US-A1- 2018 221 550

## Description

### TECHNICAL FIELD

The present invention relates to a device and to a method for the extraction of stem cells from adipose tissue.

### BACKGROUND OF THE INVENTION

As is known, stem cells are primitive, unspecialised cells, with the ability to transform into different types of cells in the body through a process called cell differentiation. They have been studied by researchers to treat certain diseases by exploiting their ductility.

Stem cells can be collected from different sources, such as the umbilical cord, amniotic sac, blood, bone marrow, placenta, and adipose tissues.

Among the various tissues listed above, adipose tissue is easily accessible and therefore forms the most convenient extraction region.

This tissue contains many types of cells, including connective cells, stroma and a small percentage (around 100) of stem cells.

For this reason, it is necessary to extract a large amount of tissue (typically 6-10 grams) to be able to proceed with extraction of the stem cells. However, in some particularly thin subjects, it is difficult to obtain this amount of tissue. US201652537 discloses a device suitable for extracting stem cells from adipose tissue, comprising a housing provided with containers for the fluids required for the operation and a robotic arm provided with a gripper adapted to engage and move the tissue processing container or any other container to the positions required to perform the different steps of the operation. The fraction with the cells is concentrated by filtration and centrifugation.

The object of the present invention is to produce a device and a method for the extraction of stem cells from a limited amount of adipose tissue, in particular an amount of adipose tissue of around 1 gram. In particular, the object of the present invention is to produce a device that operates completely automatically.

Moreover, the object of the present invention is to produce a device and a method for the extraction of stem cells that makes it possible to control the experimental variables currently dependent on the single operator and capable of obtaining, with greater probability, homogeneous cells from a phenotypic, functional and genetic point of view.

### SUMMARY OF THE INVENTION

The preceding object is achieved by the present invention, which relates to a device and to a method for the extraction of stem cells from adipose tissue as described in the appended claims.

### BRIEF DESCRIPTION OF THE DRAWINGS

The invention will now be illustrated with reference to the accompanying figures which represent a non-limiting embodiment thereof, wherein:
Fig. 1 illustrates, in a schematic view from above, a device produced according to the dictates of the present invention;
Fig. 2 illustrates a sequence of steps carried out by the present device; and
Fig. 3 illustrates a detail of the device of Fig. 1.

### DETAILED DESCRIPTION OF THE INVENTION

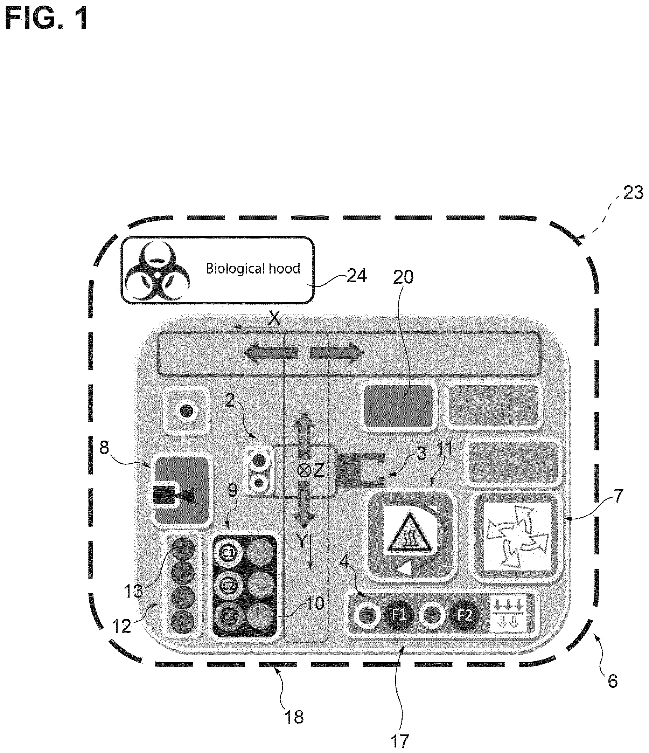

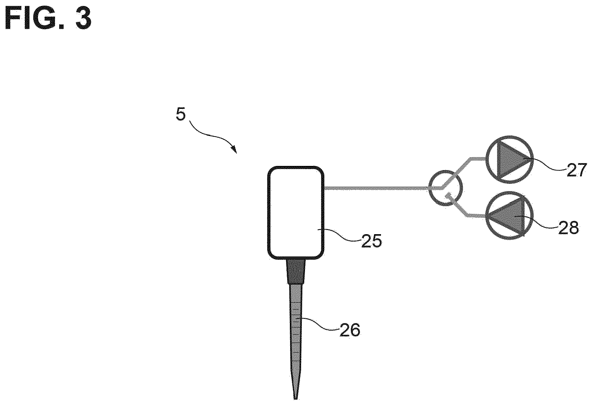

**With reference to** **Fig. 1****,** the device 1 of the present invention is provided with a robotised handling system 2 (of a known type and represented schematically) wherein a gripping element (gripper) 3 that moves in three-dimensional space along three axes X, Y and Z under the thrust of actuators is designed to collect and move a container 4 / a suction and discharge system 5 (Fig. 3) to move it between different units, which will be illustrated below. The suction / discharge system 5 is capable of managing volumes of fluid that exceed 3 ml and is also designed to aspirate and release high density fluids.

The device 1 comprises the following units: a filtration system 6 (of a known type), a heat stirrer 11 (of a known type), a centrifugation system 7 (of a known type), a viewing system 8 (of a known type), and a plurality of containers 9 for the cell culture arranged on a supporting structure (rack) 10, a rack 12 for reagents 13, a buffer station 17 in which the container 4 can be temporarily arranged.

There is provided a loading station 18 in which a container 4 to be subjected to treatment is arranged.

There is provided a single container 23 (illustrated schematically) provided with a biological hood 24 and configured to contain the robotised handling system 2, the filtration system 6, the stirrer 11, the centrifugation system 7, the culture devices, the reagents rack 12, the viewing system 8 and the buffer station 17.

With reference to Fig. 3, the suction and discharge system 5 comprises a container 25 provided, at one end thereof, with a tip 26 designed to aspirate/release fluids. The container 25 is connected with a suction system 27 to produce the vacuum in the container 25 and obtain suction of fluid in the tip 26 and with a system 28 designed to alternatively generate a pressure to produce the increase in pressure in the container 25 and obtain expulsion of the fluid contained in the tip 26.

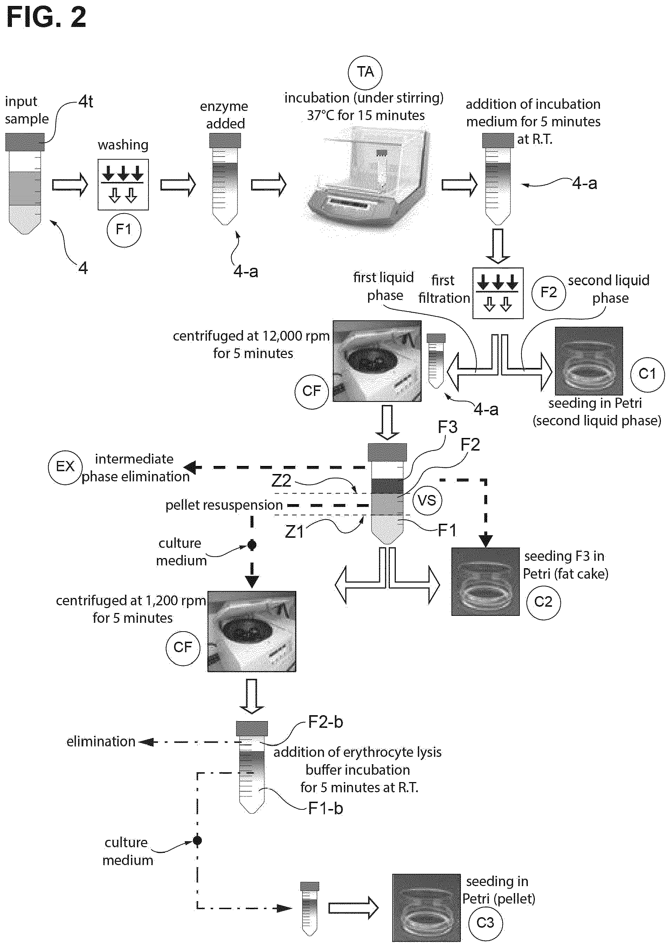

The operations of the device 1 are performed according to what is illustrated below in Fig. 2 based on controls imparted by an electronic control unit 20.

Advantageously, the adipose tissue collected from the patient is dissolved in an isotonic solution (input sample in Fig. 2) and contained in a graduated transparent tubular container 4 provided with a screw-on cap 4t. For example, the container known with the trade name Falcon 50, which can contain 50 ml of liquid, can be used. As is known, a solution is called isotonic when it has the same osmotic pressure, with respect to another solution from which it is separated through a semi-permeable membrane.

The container 4 is manually arranged on the loading station 18: subsequently, the electronic unit 20 automatically controls collection of the container 4 by means of the gripper 3 so that the container 4 is transferred to the filtration system 6, using 250 mcm nylon filters, where a first washing step with isotonic solutions is carried out by means of known techniques.

In this step, the lipoaspirate collected in the initial step is inserted (placed) on basket type nylon filters, in turn placed on Falcon tubes, and washed three times under gravity with isotonic solution.

Preferably, the washing liquid can be ejected with a highpressure jet in order to facilitate removal of blood clots and tissue debris.

The lipoaspirate, purified of the blood clots and tissue debris, is transferred from the nylon filter support into a Falcon 4-a sterile container by means of the gripper 3 controlled by the control unit 20.

The electronic control unit 20 is subsequently designed to carry out a step in which an isotonic solution containing a predetermined amount of enzyme designed to start a cell disruption process is added to the lipoaspirate. This step is advantageously carried out by arranging the container 4 in the buffer station 17 and utilising the discharge suction system 5 carried by the gripper 3 and designed to collect the enzyme from the rack 12 of the reagents 13 and release the enzyme inside the container 4 (see Fig. 2; collagenase can be used, for example, as enzyme).

The electronic control unit 20 is designed to carry out a stirring and incubation step TA in which the solution of lipoaspirate and enzyme contained in the container 4 is prewashed by the buffer station 17, supplied to the stirrer 11 where the solution of lipoaspirate and enzyme is stirred, thereby starting a chemical and mechanical breakdown process of the adipose tissue. The stirring operations take place, preferably but not exclusively, at 250 rpm and at a predetermined temperature of 37 °C corresponding to the temperature of the human body. This temperature is guaranteed by a thermoregulator (not illustrated).

The electronic unit 20 controls collection of the container 4-a from the stirrer 11 and re-transfers the container 4 to the filtration system 5 where a first filtering step is carried out, again by means of known techniques, such as using nylon filters.

A first microparticulate liquid phase containing the adipose stem cells is thus separated from a second semi-liquid phase of undigested adipose tissue.

The second semi-liquid phase of undigested adipose tissue is transferred by means of the gripper 3 into a first culture device C1 of a known type generally formed by a Petri plate or Petri capsule. This culture device C1 is arranged on the supporting rack 12 and is maintained in a controlled environment to obtain multiplication of the cells, thereby growing a first culture **C1** of adipose stem cells.

The electronic unit 13 re-transfers the container 4-a to the centrifugation system 7 that receives the first microparticulate liquid phase and subjects it to a centrifugation operation **CF** producing a product that separates into three liquid phases and precisely:
- a first denser liquid phase **F1** (pellet) that contains mainly stem cells; this phase is arranged on the bottom of the vertically oriented container 4-a:
- a second liquid phase **F2** with intermediate density that contains mainly BSA, HEPES, glucose, NaCl, KCl, CaCl₂ dissolved in water; this phase is arranged in the container 4-a on top of the first phase F1;
- a third liquid phase **F3** with lower density that contains, based on the findings of the inventors, stem cells and mature adipose cells (fat cake); this third phase is arranged in the container 4-a on top of the second phase **F2.**

The viewing system 8 detects an image of the three liquid phases arranged in the transparent container 4 and identifies on the image the separation zones Z1, Z2 between the first and second phases and the second and third phases, respectively. Knowing the separation zones Z1, Z2 allows the selective collection of the three phases, for example, by means of selective suction. This suction is carried out using the suction and discharge system 5 carried by the gripper 3.

According to the present invention, the third liquid phase F3 (also called fat cake), after having been separated from the other phases, is transferred by means of the gripper 3 into a second culture device C2 of a known type generally formed by Petri plates or Petri capsules. This second culture device **C2** is arranged on the supporting rack 12 and is maintained in a controlled environment to obtain multiplication of the stem cells thereby growing a second culture of adipose stem cells. Growth of the stem cells is controlled by means of observation, by the operator, with an optical microscope that is carried into position by the gripper 3. This microscope is associated with a viewing system for viewing images of the culture on the monitor.

After having eliminated the second liquid phase F2 with intermediate density (EX), a culture medium utilised for growth of the adipose stem cells is added to the first liquid phase F1 (pellet). The electronic unit 13 re-transfers the container containing the first liquid phase **F1** (containing pellet + culture medium) to the centrifugation system 7 and subjects it to a centrifugation operation **CF** typically carried out at 1,2000 rpm for 5 minutes at room temperature producing a product that separates into two phases and precisely:
- a further first denser liquid phase **F1-b** (pellet) that contains mainly stem cells; this phase is arranged on the bottom of the vertically oriented container 5; and
- a further second liquid phase **F2-b** with intermediate density that contains culture medium; this phase is arranged in the container 5 on top of the further first phase **F1-b.**

By means of selective suction the further second liquid phase **F2-b** is eliminated and culture medium is added to the further first liquid phase **F1-b** for resuspension of the pellet. The suspension containing the further first phase F1-b and the culture medium is transferred by the gripper 3 into a third culture device C3 of a known type, generally formed by a Petri plate or Petri capsule. This third culture device is arranged on the supporting rack 12 and is maintained in a controlled environment to obtain multiplication of the stem cells thereby growing a third culture of adipose stem cells. Also in this case, growth of the stem cells is controlled by means of observation, by the operator, with an optical microscope that is carried into position by the gripper 3.

The novelty of the present invention is represented by initiation of several cultures (three) of stem cells thereby maximising the yield of the sample collected.

In fact, the inventors of the present patent application have discovered that also the first phase, represented by undigested adipose tissue and which is normally eliminated, contains a sufficient number of stem cells to initiate a cell culture. The procedure carried out by the device illustrated above is completely automatic, as the operator requires only to arrange the sample in the loading position, after which all the operations for processing the sample are carried out by the device 1 without any human intervention. The operator only provides for collection of the culture devices.

## Claims

1. A device (1) for the extraction of stem cells from adipose tissue, wherein a robotised handling system (2) is provided with a gripper (3) that moves in three-dimensional space and is designed to collect and move a container (4) / a suction and discharge system (5) to move it between different units comprising at least a filtration system (6), a stirrer (11) and a centrifugation system (7);
an electronic control unit (20) is configured to control the performance of the following operations:
collecting by means of the gripper (3) a container (4) containing adipose tissue possibly dissolved in an isotonic solution and transferring this container (4) to the filtration system (6) where a washing step is carried out to eliminate blood clots and tissue debris;
adding to the adipose tissue subjected to washing an isotonic solution containing a predetermined amount of enzyme designed to start a cell disruption process;
supplying the solution of lipoaspirate and enzyme contained in the container (4) to the stirrer (11) to carry out a stirring step **TA** thereby starting a chemical and mechanical breakdown process of the adipose tissue;
transferring the container (4) to the filtration system (6) where a first filtering step is carried out, separating a first microparticulate liquid phase containing adipose stem cells and a second semi-liquid phase of undigested adipose tissue containing adipose stem cells;
transferring the second semi-liquid phase of undigested adipose tissue by means of the gripper (3) into a first culture device (C1) maintained in a controlled environment to obtain multiplication of the cells thereby growing a first culture (C1) of adipose stem cells;
transferring the container (4) containing the first microparticulate phase to the centrifugation system (7) that receives the first phase and subjects it to a centrifugation operation (**CF**) producing a product that separates inside the container into three phases and precisely:
• a first denser liquid phase (F1) (pellet) that contains mainly stem cells - this first liquid phase (F1) is arranged on the bottom of a vertically oriented container;
• a second liquid phase (F2) with intermediate density that contains mainly BSA, HEPES,glucose, NaCl, KCl, CaCl₂ dissolved in water - this second liquid phase (F2) is arranged in the container on top of the first liquid phase (F1);
• a third liquid phase (fat cake; F3) with lower density that contains stem cells and mature adipose cells - this third phase is arranged in the container on top of the second liquid phase (F2) ;
separating the third phase (F3) from the other phases by means of the suction and discharge system (5) carried by the gripper (3) and placing it in a second culture device C2 that is maintained in a controlled environment to obtain multiplication of the stem cells thereby growing a second cell culture (C2); and
separating the first phase (F1) from the other phases by means of the suction and discharge system and adding a culture medium to the first liquid phase (F1);
the electronic unit (13) is configured to transfer the container containing the first phase containing the culture medium to the centrifugation system (7) that subjects the first phase to a centrifugation operation (**CF)** producing a product that separates into two further liquid phases and precisely:
• a further denser first phase (F1-b) (pellet) that contains mainly stem cells - this further first phase is arranged on the bottom of the vertically oriented container (4);
• a further second phase (F2-b) with intermediate density that contains the culture medium - this further second phase is arranged in the container (4) on top of the first phase (F1);
the electronic unit is designed to control the selective suction of the second further phase (F2-b) that is eliminated and is designed to add a culture medium to the further first phase (F1-b) for resuspension of the pellet;
the electronic unit is designed to control the transfer by means of gripper of the suspension containing the pellet into a third culture device (C3) maintained in a controlled environment to obtain multiplication of the stem cells thereby growing a third culture of adipose stem cells.

2. The device according to claim 1, wherein there is provided a viewing system (8) configured to detect an image of the three phases arranged in the transparent container (4) and identifying on the image the separation zones Z1, Z2 between the first and the second phase and the second and the third phase, respectively; said electronic unit (20) being configured to control the selective collection of the three phases, for example by means of selective suction, based on the information on the separation zones.

3. The device according to any one of the independent claims, wherein there is provided a single container (23) provided with a biological hood (24) and configured to contain the robotised handling system (2), the filtration system (6), the stirrer (11), the centrifugation system (7) and the culture devices.

4. The device according to one of the preceding claims, wherein said suction and discharge system (5) comprises a container (25) provided, at one end thereof, with a tip (26) configured to aspirate/release fluids; said container (25) is connected with a suction system (27) to produce the vacuum in the container (25) and obtain suction of fluid in the tip (26) and with a system (28) designed to generate a pressure to produce the increase in pressure in the container (25) and obtain expulsion of the fluid contained in the tip (26).

5. A method for the extraction of stem cells from adipose tissue comprising the following operations:
collecting a container (4) containing adipose tissue possibly dissolved in an isotonic solution and transferring this container (4) onto a filter to carry out a washing step of the lipoaspirate;
adding to the lipoaspirate subjected to washing a solution containing a predetermined amount of enzyme designed to start a cell disruption process;
supplying the solution of lipoaspirate and enzyme contained in the container (4-a) to a stirrer (11) to carry out a stirring step **TA** thereby starting a chemical and mechanical breakdown process of the adipose tissue;
transferring the container (4) to the filtration system (6) where a first filtering phase is carried out, separating a first microparticulate liquid phase containing adipose stem cells and a second semi-liquid phase containing undigested adipose tissue;
supplying the second semi-liquid phase of undigested adipose tissue to a first culture device (C1) maintained in a controlled environment to obtain multiplication of the cells thereby growing a first culture (C1) of adipose stem cells;
transferring the container (4) containing the first microparticulate phase to the centrifugation system (7) that receives the first microparticulate liquid phase and subjects it to a centrifugation operation (**CF**) producing a product that separates inside the container into three phases and precisely:
• a first denser liquid phase (F1) (pellet) that contains mainly stem cells - this phase is arranged on the bottom of a vertically oriented container:
• a second liquid phase (F2) with intermediate density that contains mainly BSA, HEPES,glucose, NaCl, KCl, CaCl₂ dissolved in water - this phase is arranged in the container on top of the first phase (F1);
• a third liquid phase (fat cake; F3) with lower density that contains stem cells and mature adipocytes - this third liquid phase (F3) is arranged in the container on top of the second liquid phase (F2);
separating the third liquid phase (F3) from the other phases and placing it in a second culture device (C2) that is maintained in a controlled environment to obtain multiplication of the stem cells thereby growing a second cell culture (C2); and
separating the first liquid phase (F1) from the other phases and adding a culture medium to the first liquid phase (F1);
subjecting the first liquid phase (F1) containing the culture medium to a centrifugation operation **(CF)** producing a product that separates into two further phases and precisely:
• a further first denser liquid phase (F1-b) that contains mainly stem cells - this phase is arranged on the bottom of the vertically oriented container (5);
• a further second liquid phase (F2-b) with intermediate density that contains the culture medium - this further second phase (F2-b) is arranged in the container (5) on top of the further first phase (F1-b);
eliminating by means of selective suction the further second liquid phase (F2-b) and adding a culture medium to the further first phase (F1-b) for resuspension of the pellet;
transferring the suspension containing the pellet into a third culture device (C3) maintained in a controlled environment to obtain multiplication of the stem cells thereby growing a third culture (C3) of adipose stem cells.

6. The method according to claim 5, wherein there is provided the step of detecting an image of the three phases arranged in the transparent container (4) and identifying on the image the separation zones Z1, Z2 between the first and the second phase and the second and the third phase, respectively; selectively collecting the three phases, for example by means of selective suction, based on the information on the separation zones.

## Patentansprüche

1. Vorrichtung (1) für die Extraktion von Stammzellen aus Fettgewebe, wobei ein robotisiertes Handlingsystem (2) mit einem im dreidimensionalen Raum beweglichen Greifer (3) ausgestattet ist, der zur Aufnahme und Bewegung eines Behälters (4)/ einem Ansaug- und Abführsystem (5) ausgestaltet ist, um ihn/es zwischen verschiedenen Einheiten zu bewegen, die mindestens ein Filtrationssystem (6), einen Rührer (11) und ein Zentrifugationssystem (7) umfassen;
eine elektronische Steuereinheit (20), die konfiguriert ist, die Durchführung der folgenden Vorgänge zu steuern:
Aufnehmen mithilfe des Greifers (3) eines Behälters (4), der Fettgewebe enthält, das möglicherweise in einer isotonischen Lösung gelöst ist, und Überführen des Behälters (4) in das Filtrationssystem (6), wo ein Waschschritt durchgeführt wird, um Blutgerinnsel und Gewebetrümmer zu entfernen;
Hinzufügen einer isotonischen Lösung, die eine vorbestimmte Menge an Enzym enthält, zu dem dem Waschen unterzogenen Fettgewebe, um einen Zellaufschlussprozess zu starten;
Zuführen der Lösung von Lipoaspirat und Enzym, die im Behälter (4) enthalten ist, in den Rührer (11), um einen Rührschritt **TA** durchzuführen, wodurch ein chemischer und mechanischer Abbauprozess des Fettgewebes gestartet wird;
Überführen des Behälters (4) in das Filtrationssystem (6), wo ein erster Filterschritt durchgeführt wird, wobei eine erste flüssige Mikropartikelphase, die Fettstammzellen enthält, und eine zweite halbflüssige Phase von unverdautem Fettgewebe, die Fettstammzellen enthält, getrennt werden;
Überführen der zweiten halbflüssigen Phase unverdauten Fettgewebes mithilfe des Greifers (3) in eine erste Kulturvorrichtung (C1) , die in einer kontrollierten Umgebung gehalten wird, um eine Vermehrung der Zellen zu erreichen, wodurch eine erste Kultur (C1) von Fettstammzellen gezüchtet wird;
Überführen des Behälters (4), der die erste Mikropartikelphase enthält, in das Zentrifugationssystem (7), das die erste Phase aufnimmt und sie einem Zentrifugationsvorgang (**CF**) unterzieht, wobei ein Produkt hergestellt wird, das im Innern des Behälters in drei Phasen getrennt ist, und zwar genauer:
• eine erste dichtere flüssige Phase (F1) (Pellet), die hauptsächlich Stammzellen enthält, wobei diese erste flüssige Phase (F1) auf dem Boden eines vertikal ausgerichteten Behälters angeordnet ist;
• eine zweite flüssige Phase (F2) mit mittlerer Dichte, die hauptsächlich in Wasser gelöstes BSA, HEPES, Glucose, NaCl, KCl, CaCl₂ enthält, wobei diese zweite flüssige Phase (F2) in dem Behälter auf der ersten flüssigen Phase (F1) angeordnet ist;
• eine dritte flüssige Phase (Fettkuchen; F3) mit einer geringeren Dichte, die Stammzellen und reife Fettzellen enthält, wobei diese dritte Phase in dem Behälter auf der zweiten flüssigen Phase (F2) angeordnet ist;
Trennen der dritten Phase (F3) von den anderen Phasen mithilfe des Ansaug- und Abführsystems (5), das vom Greifer (3) getragen wird, und Platzieren dieser in einer zweiten Kulturvorrichtung (C2), die in einer kontrollierten Umgebung gehalten wird, um eine Vermehrung der Stammzellen zu erreichen, wodurch eine zweite Zellkultur (C2) gezüchtet wird;
und
Trennen der ersten Phase (F1) von den anderen Phasen mithilfe des Ansaug- und Abführsystems und Hinzufügen eines Kulturmediums zu der ersten flüssigen Phase (F1);
wobei die elektronische Einheit (13) konfiguriert ist, um den Behälter, der die erste das Kulturmedium enthaltende Phase enthält, in das Zentrifugationssystem (7) zu überführen, das die erste Phase einem Zentrifugationsvorgang **(CF)** unterzieht, der ein Produkt erzeugt, das in zwei weitere flüssige Phasen getrennt ist, und zwar genauer:
• eine weitere dichtere erste Phase (F1-b) (Pellet), die hauptsächlich Stammzellen enthält, wobei die weitere erste Phase auf dem Boden des vertikal ausgerichteten Behälters (4) angeordnet ist;
• eine weitere zweite Phase (F2-b) mit mittlerer Dichte, die das Kulturmedium enthält, wobei die weitere zweite Phase in dem Behälter (4) auf der ersten Phase (F1) angeordnet ist;
wobei die elektronische Einheit ausgestaltet ist, das selektive Ansaugen der zweiten weiteren Phase (F2-b) zu steuern, die entfernt wird, und die ausgestaltet ist, der weiteren ersten Phase (F1-b) ein Kulturmedium zur Resuspension des Pellets hinzuzufügen;
wobei die elektronische Einheit ausgestaltet ist, die Überführung der Suspension, die das Pellet enthält, mithilfe des Greifers in eine dritte Kulturvorrichtung (C3) zu steuern, die in einer kontrollierten Umgebung gehalten wird, um eine Vermehrung der Stammzellen zu erreichen, wodurch eine dritte Kultur von Fettstammzellen gezüchtet wird.

2. Vorrichtung nach Anspruch 1, wobei ein Sichtsystem (8) bereitgestellt ist, das konfiguriert ist, um ein Bild der drei in dem transparenten Behälter (4) angeordneten Phasen zu erfassen und auf dem Bild die Trennungszonen Z1, Z2 zwischen der ersten und der zweiten Phase bzw. der zweiten und der dritten Phase zu identifizieren; wobei die elektronische Einheit (20) so konfiguriert ist, dass sie die selektive Aufnahme der drei Phasen, zum Beispiel mithilfe des selektiven Ansaugens, auf der Grundlage der Information über die Trennungszonen steuert.

3. Vorrichtung nach einem der unabhängigen Ansprüche, wobei ein einzelner Behälter (23) bereitgestellt ist, der mit einer biologischen Haube (24) versehen ist und konfiguriert ist, das robotisierte Handlingsystem (2), das Filtrationssystem (6), den Rührer (11), das Zentrifugationssystem (7) und die Kulturvorrichtungen zu enthalten.

4. Vorrichtung nach einem der vorangehenden Ansprüche, wobei das Ansaug- und Abführsystem (5) einen Behälter (25) umfasst, der an einem seiner Enden mit einer Spitze (26) versehen ist, die konfiguriert ist, Flüssigkeiten anzusaugen/freizusetzen; wobei der Behälter (25) mit einem Saugsystem (27) verbunden ist, um das Vakuum in dem Behälter (25) herzustellen und das Ansaugen von Flüssigkeit in der Spitze (26) zu erreichen, und mit einem System (28), das ausgestaltet ist, einen Druck zu erzeugen, um den Druckanstieg in dem Behälter (25) zu erzeugen und den Ausstoß der in der Spitze (26) enthaltenen Flüssigkeit zu erreichen.

5. Verfahren zur Extraktion von Stammzellen aus Fettgewebe, umfassend die folgenden Vorgänge:
Aufnehmen eines Behälters (4), der Fettgewebe enthält, das möglicherweise in einer isotonischen Lösung gelöst ist, und Überführen dieses Behälters (4) auf einen Filter, um einen Waschschritt des Lipoaspirats durchzuführen;
Hinzufügen einer Lösung, die eine vorbestimmte Menge an Enzym enthält, zu dem Lipoaspirat, das dem Waschen unterzogen wurde, das dazu bestimmt ist, einen Zellaufschlussprozess zu starten;
Zuführen der Lösung von Lipoaspirat und Enzym, die in dem Behälter (4-a) enthalten ist, in einen Rührer (11), um einen Rührschritt **TA** durchzuführen, wobei ein chemischer und mechanischer Abbauprozess des Fettgewebes gestartet wird;
Überführen des Behälters (4) in das Filtrationssystem (6), wo ein erster Filterschritt durchgeführt wird, wobei eine erste flüssige Mikropartikelphase, die Fettstammzellen enthält, und eine zweite halbflüssige Phase, die unverdautes Fettgewebe enthält, getrennt werden;
Zuführen der zweiten halbflüssigen Phase unverdauten Fettgewebes in eine erste Kulturvorrichtung (C1), die in einer kontrollierten Umgebung gehalten wird, um eine Vermehrung der Zellen zu erreichen, wodurch eine erste Kultur (C1) von Fettstammzellen gezüchtet wird;
Überführen des Behälters (4), der die erste Mikropartikelphase enthält, in das Zentrifugationssystem (7), das die erste flüssige Mikropartikelphase aufnimmt und sie einem Zentrifugationsvorgang (**CF**) unterzieht, wobei ein Produkt erzeugt wird, das im Innern des Behälters in drei Phasen getrennt ist, und zwar genauer:
• eine erste dichtere flüssige Phase (F1) (Pellet), die hauptsächlich Stammzellen enthält, wobei diese Phase auf dem Boden eines vertikal ausgerichteten Behälters angeordnet ist;
• eine zweite flüssige Phase (F2) mit mittlerer Dichte, die hauptsächlich in Wasser gelöstes BSA, HEPES, Glucose, NaCl, KCl, CaCl₂ enthält, wobei diese Phase in dem Behälter auf der ersten Phase (F1) angeordnet ist;
• eine dritte flüssige Phase (Fettkuchen; F3) mit einer geringeren Dichte, die Stammzellen und reife Adipocyten enthält, wobei diese dritte flüssige Phase in dem Behälter auf der zweiten flüssigen Phase (F2) angeordnet ist;
Trennen der dritten flüssigen Phase (F3) von den anderen Phasen und Platzieren dieser in einer zweiten Kulturvorrichtung (C2), die in einer kontrollierten Umgebung gehalten wird, um eine Vermehrung der Stammzellen zu erreichen, wodurch eine zweite Zellkultur (C2) gezüchtet wird; und
Trennen der ersten flüssigen Phase (F1) von den anderen Phasen und Hinzufügen eines Kulturmediums zu der ersten flüssigen Phase (F1);
Unterziehen der ersten flüssigen Phase (F1), die das Kulturmedium enthält, einem Zentrifugationsvorgang (**CF**), wobei ein Produkt hergestellt wird, das in zwei weitere Phasen getrennt ist, und zwar genau:
• eine weitere erste dichtere flüssige Phase (F1-b), die hauptsächlich Stammzellen enthält, wobei diese Phase auf dem Boden des vertikal ausgerichteten Behälters (5) angeordnet ist;
• eine weitere zweite flüssige Phase (F2-b) mit mittlerer Dichte, die das Kulturmedium enthält, wobei diese weitere zweite Phase (F2-b) im Behälter (5) auf der weiteren ersten Phase (F1-b) angeordnet ist;
Entfernen mithilfe selektiven Ansaugens der weiteren zweiten flüssigen Phase (F2-b) und Hinzufügen eines Kulturmediums zu der weiteren ersten Phase (F1-b) zur Resuspension des Pellets;
Überführen der Suspension, die das Pellet enthält, in eine dritte Kulturvorrichtung (C3), die in einer kontrollierten Umgebung gehalten wird, um eine Vermehrung der Stammzellen zu erreichen, wodurch eine dritte Kultur (C3) von Fettstammzellen gezüchtet wird.

6. Verfahren nach Anspruch 5, wobei der Schritt des Erfassens eines Bildes der drei in dem transparenten Behälter (4) angeordneten Phasen und Identifizieren der Trennungszonen Z1, Z2 zwischen der ersten und der zweiten Phase bzw. der zweiten und der dritten Phase auf dem Bild; des selektiven Aufnehmens der drei Phasen, zum Beispiel mithilfe selektiven Ansaugens, auf der Grundlage der Information über die Trennungszonen vorgesehen ist.

## Revendications

1. Dispositif (1) pour l'extraction de cellules souches à partir de tissu adipeux, dans lequel un système de manipulation robotisé (2) est doté d'un organe de préhension (3) qui se déplace dans l'espace tridimensionnel et est conçu pour collecter et déplacer un récipient (4) / un système d'aspiration et de décharge (5) pour le déplacer entre différentes unités comprenant au moins un système de filtration (6), un agitateur (11) et un système de centrifugation (7) ;
une unité de commande électronique (20) est configurée pour commander l'exécution des opérations suivantes :
la collecte, au moyen de l'organe de préhension (3), d'un récipient (4) contenant du tissu adipeux éventuellement dissous dans une solution isotonique, et le transfert de ce récipient (4) jusqu'au système de filtration (6) où une étape de lavage est effectuée pour éliminer les caillots sanguins et les débris tissulaires ;
l'addition, au tissu adipeux ayant subi le lavage, d'une solution isotonique contenant une quantité prédéterminée d'enzyme conçue pour initier un processus de désintégration cellulaire ;
la fourniture de la solution de lipoaspirat et d'enzyme contenue dans le récipient (4) à l'agitateur (11) pour la mise en oeuvre d'une étape d'agitation TA initiant ainsi un processus de rupture chimique et mécanique du tissu adipeux ;
le transfert du récipient (4) jusqu'au système de filtration (6) où une première étape de filtration est effectuée, la séparation d'une première phase liquide microparticulaire contenant des cellules souches adipeuses et d'une deuxième phase semi-liquide de tissu adipeux non digéré contenant des cellules souches adipeuses ;
le transfert de la deuxième phase semi-liquide de tissu adipeux non digéré, au moyen de l'organe de préhension (3), dans un premier dispositif de culture (C1) maintenu dans un environnement contrôlé pour que soit obtenue une multiplication des cellules faisant ainsi croître une première culture (C1) de cellules souches adipeuses ;
le transfert du récipient (4) contenant la première phase microparticulaire jusqu'au système de centrifugation (7) qui reçoit la première phase et la soumet à une opération de centrifugation (CF) produisant un produit qui se sépare à l'intérieur du récipient en trois phases et précisément en :
• une première phase liquide plus dense (F1) (culot) qui contient principalement des cellules souches - cette première phase liquide (F1) est disposée au fond d'un récipient orienté verticalement ;
• une deuxième phase liquide (F2) de densité intermédiaire qui contient principalement de la BSA, du HEPES, du glucose, du NaCl, du KCl, du CaCl₂, dissous dans de l'eau - cette deuxième phase liquide (F2) est disposée dans le récipient au-dessus de la première phase liquide (F1) ;
• une troisième phase liquide (gâteau gras ; F3) ayant une densité moindre qui contient des cellules souches et des cellules adipeuses matures - cette troisième phase est disposée dans le récipient au-dessus de la deuxième phase liquide (F2) ;
la séparation de la troisième phase (F3) d'avec les autres phases au moyen du système d'aspiration et de décharge (5) porté par l'organe de préhension (3) et son placement dans un deuxième dispositif de culture C2 qui est maintenu dans un environnement contrôlé pour que soit obtenue une multiplication des cellules souches faisant ainsi croître une deuxième culture cellulaire (C2) ; et
la séparation de la première phase (F1) d'avec les autres phases au moyen du système d'aspiration et de décharge et l'addition d'un milieu de culture à la première phase liquide (F1) ;
l'unité électronique (13) est configurée pour transférer le récipient contenant la première phase contenant le milieu de culture jusqu'au système de centrifugation (7) qui soumet la première phase à une opération de centrifugation (CF) produisant un produit qui se sépare en deux autres phases liquides et précisément en :
• une autre première phase plus dense (F1-b) (culot) qui contient principalement des cellules souches - cette autre première phase est disposée au fond du récipient orienté verticalement (4) ;
• une autre deuxième phase (F2-b) de densité intermédiaire qui contient le milieu de culture - cette autre deuxième phase est disposée dans le récipient (4) au-dessus de la première phase liquide (F1) ;
l'unité électronique est conçue pour commander l'aspiration sélective de l'autre deuxième phase (F2-b) qui est éliminée et est conçue pour ajouter un milieu de culture à l'autre première phase (F1-b) pour une remise en suspension du culot ;
l'unité électronique est conçue pour commander le transfert, au moyen de l'organe de préhension, de la suspension contenant le culot dans un troisième dispositif de culture (C3) maintenu dans un environnement contrôlé pour que soit obtenue une multiplication des cellules souches faisant ainsi croître une troisième culture de cellules souches adipeuses.

2. Dispositif selon la revendication 1, dans lequel il est prévu un système de visualisation (8) configuré pour détecter une image des trois phases disposées dans le récipient transparent (4) et identifiant sur l'image les zones de séparation Z1, Z2 entre les première et deuxième phases et les deuxième et troisième phases, respectivement ; ladite unité électronique (20) étant configurée pour commander la collecte sélective des trois phases, par exemple au moyen d'une aspiration sélective, sur la base des informations sur les zones de séparation.

3. Dispositif selon l'une quelconque des revendications indépendantes, dans lequel il est prévu un seul récipient (23) doté d'une hotte biologique (24) et configuré pour contenir le système de manipulation robotisé (2), le système de filtration (6), l'agitateur (11), le système de centrifugation (7) et les dispositifs de culture.

4. Dispositif selon l'une des revendications précédentes, dans lequel ledit système d'aspiration et de décharge (5) comprend un récipient (25) doté, à une extrémité de celui-ci, d'une pointe (26) configurée pour aspirer/libérer des fluides ; ledit récipient (25) est connecté à un système d'aspiration (27) pour produire le vide dans le récipient (25) et pour que soit obtenue une aspiration de fluide dans la pointe (26), et à un système (28) conçu pour générer une pression pour produire l'augmentation de pression dans le récipient (25) et pour que soit obtenue l'expulsion du fluide contenu dans la pointe (26).

5. Procédé pour l'extraction de cellules souches à partir de tissu adipeux, comprenant les opérations suivantes :
collecte d'un récipient (4) contenant du tissu adipeux éventuellement dissous dans une solution isotonique, et transfert de ce récipient (4) jusqu'à un filtre pour la mise en oeuvre d'une étape de lavage du lipoaspirat ;
addition au lipoaspirat ayant subi le lavage d'une solution contenant une quantité prédéterminée d'enzyme conçue pour initier un processus de désintégration cellulaire ;
fourniture de la solution de lipoaspirat et d'enzyme contenue dans le récipient (4-a) à un agitateur (11) pour la mise en oeuvre d'une étape d'agitation TA initiant ainsi un processus de rupture chimique et mécanique du tissu adipeux ;
transfert du récipient (4) jusqu'au système de filtration (6) où une première phase de filtration est effectuée, séparation d'une première phase liquide microparticulaire contenant des cellules souches adipeuses et d'une deuxième phase semi-liquide contenant du tissu adipeux non digéré ;
fourniture de la deuxième phase semi-liquide de tissu adipeux non digéré à un premier dispositif de culture (C1) maintenu dans un environnement contrôlé pour que soit obtenue une multiplication des cellules faisant ainsi croître une première culture (C1) de cellules souches adipeuses ;
transfert du récipient (4) contenant la première phase microparticulaire jusqu'au système de centrifugation (7) qui reçoit la première phase liquide particulaire et la soumet à une opération de centrifugation (CF) produisant un produit qui se sépare à l'intérieur du récipient en trois phases et précisément en :
• une première phase liquide plus dense (F1) (culot) qui contient principalement des cellules souches - cette phase est disposée au fond d'un récipient orienté verticalement ;
• une deuxième phase liquide (F2) de densité intermédiaire qui contient principalement de la BSA, du HEPES, du glucose, du NaCl, du KCl, du CaCl₂, dissous dans de l'eau - cette phase est disposée dans le récipient au-dessus de la première phase (F1) ;
• une troisième phase liquide (gâteau gras ; F3) ayant une densité moindre qui contient des cellules souches et des cellules adipeuses matures - cette troisième phase liquide (F3) est disposée dans le récipient au-dessus de la deuxième phase liquide (F2) ;
séparation de la troisième phase liquide (F3) d'avec les autres phases et placement de celle-ci dans un deuxième dispositif de culture (C2) qui est maintenu dans un environnement contrôlé pour que soit obtenue une multiplication des cellules souches faisant ainsi croître une deuxième culture cellulaire (C2) ; et
séparation de la première phase liquide (F1) d'avec les autres phases et addition d'un milieu de culture à la première phase liquide (F1) ;
soumission de la première phase liquide (F1) contenant le milieu de culture à une opération de centrifugation (CF) produisant un produit qui se sépare en deux autres phases liquides et précisément en :
• une autre première phase liquide plus dense (F1-b) qui contient principalement des cellules souches - cette phase est disposée au fond du récipient orienté verticalement (5) ;
• une autre deuxième phase liquide (F2-b) de densité intermédiaire qui contient le milieu de culture - cette autre deuxième phase (F2-b) est disposée dans le récipient (5) au-dessus de la première phase liquide (F1-b) ;
élimination, au moyen d'une aspiration sélective, de l'autre deuxième phase liquide (F2-b) et addition d'un milieu de culture à l'autre première phase (F1-b) pour une remise en suspension du culot ;
transfert de la suspension contenant le culot dans un troisième dispositif de culture (C3) maintenu dans un environnement contrôlé pour que soit obtenue une multiplication des cellules souches faisant ainsi croître une troisième culture (C3) de cellules souches adipeuses.

6. Procédé selon la revendication 5, dans lequel il est prévu une étape de détection d'une image des trois phases disposées dans le récipient transparent (4) et d'identification sur l'image des zones de séparation Z1, Z2 entre les première et deuxième phases et les deuxième et troisième phases, respectivement ; et la collecte sélective des trois phases, par exemple au moyen d'une aspiration sélective, sur la base des informations sur les zones de séparation.
